# EUROPEAN PATENT APPLICATION

(11) **EP 4 140 516 A1**
(43) Date of publication of application: **01.03.2023**
(21) Application number: 21020435.0
(22) Date of filing: 31.08.2021
(51) Int. Cl.: A61M 1/02, A61M 5/14, H02J 50/00, A61B 5/15, A61M 5/165

(54) **A BLOOD TRANSFUSION SET DESIGN INNOVATION FOR SHORTENING TREATMENT TIME AND AVOIDING MULTIPLE PUNCTURES TO PATIENTS**

(71) Applicant: LundaTec AB, 223 63 Lund (SE)
(72) Inventor: The designation of the inventor has not yet been filed

(57) **Abstract**

The utility model provides a blood transfusion set comprising a main line, which is connected with the first branch line and the second branch line; the first branch line is connected with the third branch line and the fourth branch line; the free end of the main line is provided with an infusion needle; the free ends of the second branch line, the third branch line and the fourth branch line are all provided with spikes; the first branch line, the second branch line, the third branch line and the fourth branch line are all provided with clamps. The utility model can draw blood through the second branch line, and perform infusion through the third branch line or transfusion through the fourth branch line without replacing the infusion needle. It not only shortens treatment time, but also avoid multiple punctures in patients.

## Description

### Technical Field

This utility model relates to the field of medical device, to a blood transfusion set.

### Background Art

At present, hospitals often separately use blood taking sets, blood transfusion sets, and infusion sets when performing surgeries on patients. In reality, many patients need to draw blood for testing first, then receive infusion and transfusion, as well as intermittent infusion after transfusion. Therefore, it is necessary to frequently replace blood taking sets, blood transfusion sets and infusion sets, which not only delays the treatment, but also makes the patient suffer from multiple punctures.

### Disclosure of Invention

In view of this, the utility model provides a blood transfusion set, which aims to solve the problem of oversimplified function of the blood transfusion set.

In order to achieve the above purpose, the technical solution adopted by the utility model is:
A blood transfusion set comprises a main line, which is connected with the first branch line and the second branch line; the first branch line is connected with the third branch line and
the fourth branch line; the free end of the main line is provided with an infusion needle; the free ends of the second branch line, the third branch line, and the fourth branch line are all provided with spikes; the first branch line, the second branch line, the third branch line and
the fourth branch line are all provided with clamps.

Furthermore, the main line comprises the first infusion line and the second infusion line; one end of the first infusion line is connected with an infusion needle, and the other end of the first infusion line is provided with the first line connector; one end of the second infusion line is provided with the second line connector, and the other end of the second infusion line is connected with the first branch line and the second branch line.

Furthermore, the first line connector and the second line connector are detachably connected together; the first infusion line is connected with the second infusion line.

Furthermore, the main line, the first branch line, the second branch line, the third branch line and the fourth branch line are made of PVC material with the same diameter.

Furthermore, the second branch line is provided with a flow regulator, a drip chamber and a filter; the clamp, flow regulator, drip chamber and filter in the second branch line are set in sequence from the end of the second branch line connected with the main line to the other end of the second branch line.

Furthermore, the filter is provided with a filter screen; the filter is connected to the drip chamber through a drip tube.

Furthermore, the main line, the first branch line and the second branch line are connected together through a T valve.

Furthermore, the first branch line, the third branch line and the fourth branch line are connected together through a T valve.

Furthermore, the blood transfusion set comprises an illuminated sensor which is detachably provided at the drip chamber.

Furthermore, the illuminated sensor comprises an LED lamp and an energy converter; the energy converter is electrically connected to the LED lamp.

Compared with the prior art, this utility model can draw blood through the second branch line, and perform infusion through the third branch line or transfusion through the fourth branch line without replacing the infusion needle. It not only shortens treatment time, but also avoid multiple punctures in patients.

### Brief Description of Drawings

The attached drawings as a part of the specification, are provided for a further understanding of this utility model. The exemplary embodiments and descriptions are used to describe the utility model, and do not constitute improper limitations to the utility model. In the attached drawings:
Drawing 1 is a structural diagram of a mode of execution of infusion set;
Drawing 2 is a partial enlarged drawing of A in Drawing 1;
Drawing 3 is a structural diagram of a mode of execution of energy converter;
Drawing 4 is a structural diagram of positive charge induction collector in Drawing 3;
Drawing 5 is a partial enlarged drawing of II in Drawing 4;
Drawing 6 is a structural diagram of negative charge induction collector in Drawing 3;
Drawing 7 is a partial enlarged drawing of I in Drawing 6;
Drawing 8 is a structural diagram of positive charge induction collector mated with negative charge induction collector in Drawing 3;
Drawing 9 is a structural diagram of positive plate and negative plate provided with a discharge ring.

### Description of Drawing Marks:

1-Main Line, 2-Second Branch Line, 3-First Branch Line, 4-Third Branch Line, 5-Fourth Branch Line, 6-Clamp, 7-Spike, 8-Energy Converter;
11-Infusion Needle, 12-First Infusion Line, 13-Second Infusion Line, 31-Flow Regulator, 32-Drip Chamber, 33-Filter, 81-Outer Frame, 82-Piezoelectric Device, 83-Positive Charge Induction Collector, 84-Negative Charge Induction Collector, 85-Pulling Rope, 86-Through Hole, 87-Discharge Ring, 88-Outer Plate;
121-First Line Connector, 131-Second Line Connector, 831-Positive Plate, 832-Positive Pulling Structure, 833-Positive Rectifier Anchoring Structure, 834-Positive Piezoelectric Structure, 835-Forward Diode, 836-Positive Anchoring Structure; 841-Negative Plate, 842-Negative Pulling Structure, 843-Negative Rectifying Anchoring Structure, 844-Negative Piezoelectric Structure, 845-Backward Diode, 846-Negative Anchoring Structure.

### Best Mode(s) for Carrying out the Invention

It should be noted the embodiments in the present disclosure and the features in the embodiments can be combined with each other without conflict. Moreover, the terms in the embodiments such as "length", "width", "thickness", "upper", "lower", "front", "rear", "left", "right", "vertical", "horizontal", "top", "bottom", "inner" and "outer" indicate the orientation or position based on the attached drawings. It is only intended to facilitate and simplify the description, rather than indicate or imply that the device or element must have a specific orientation or be constructed and operated in a specific orientation. Therefore, it cannot be understood as a limitation of the present invention.

Hereinafter, embodiments of the present invention will be described in detail with reference to the accompanying drawings.

### Embodiment 1

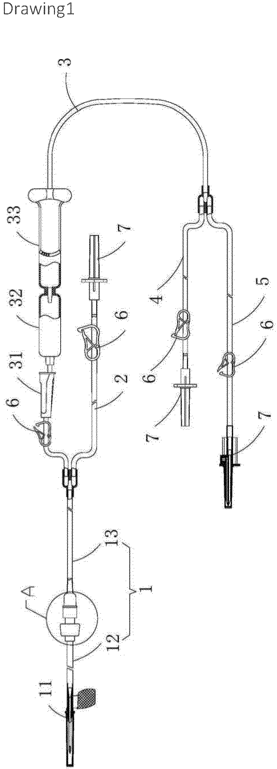

As shown in Drawing 1, a blood transfusion set comprises a main line (1), which is connected with the first branch line (3) and the second branch line (2); the first branch line (3) is connected with the third branch line (4) and the fourth branch line (5); the free end of the main line (1) is provided with an infusion needle (11); the free ends of the second branch line (2), the third branch line (4), and the fourth branch line (5) are all provided with spikes (7); the first branch line (3), the second branch line (2), the third branch line (4) and the fourth branch line (5) are all provided with clamps (6).

In this embodiment, the first branch line (3) and the third branch line (4) are occluded by respective clamps (6), and the second branch line (2) and the fourth branch line (5) are released by clamps; the fourth branch line (5), the second branch line (2) and the main line (1) are used together to clean the tube or for infusion. Specifically, the spike (7) of the fourth branch line (5) is connected to the infusion bottle. Under negative pressure, the liquid in the infusion bottle flows out along the fourth branch line (5), the second branch line (2) and the main line (1) to clean the tube when the infusion needle (11) is not inserted into the blood vessel or perform infusion when the infusion needle (11) is inserted into the blood vessel.

In this embodiment, the second branch line (2), the third branch line (4), and the fourth branch line (5) are occluded by their respective clamps (6), and the clamp (6) of the first branch line (3) is used to release; the first branch line (3) cooperates with the main line (1) to collect blood, specifically, the spike (7) of the first branch line (3) is connected to a vacuum blood bag, after the infusion needle (11) of the main line (1) is inserted into the human body, the blood enters the vacuum blood bag through the main line (1) and the first branch line (3) under negative pressure; clean the main line (1) by the above-mentioned method prior to blood sampling.

In this embodiment, the first branch line (3), the fourth branch line (5) are occluded by their respective clamps (6), and the clamp of the second branch line (2) and the third branch line (4) is used to release; the third branch line (4), the second branch line (2), and the main line (1) cooperate for blood transfusion, specifically, the spike (7) of the third branch line (4) is connected to the blood bag, the blood in the blood bag outflows along the third branch line (4), the second branch line (2), and the main line (1) under negative pressure, and the blood is transfused when the infusion needle (11) is inserted into the blood vessel; clean the main line (1) and the third branch line (4) by the above-mentioned method prior to blood transfusion.

In this embodiment, the main line (1), the first branch line (3), the second branch line (2), the third branch line (4), and the fourth branch line (5) are made of PVC and have equal bore diameters. Therefore, it is convenient to observe and transfer liquid and blood.

In this embodiment, the main line (1), the first branch line (3), and the second branch line (2) are connected together by a T valve; The first branch line 3, the third branch line (4), and the fourth branch line (5) are connected together by a T valve, thereby facilitating the communication between the lines.

In this embodiment, the infusion set further includes illuminated sensors, which are detachably installed at the drip chamber (32). Specifically, the illuminated sensors include LED lamp and energy converter (8), which are glued to the drip chamber (32). The energy converter (8) is electrically connected to an LED lamp, so that the LED light can be used in a dim condition to check the liquid level of the drip chamber (32).

### Embodiment 2

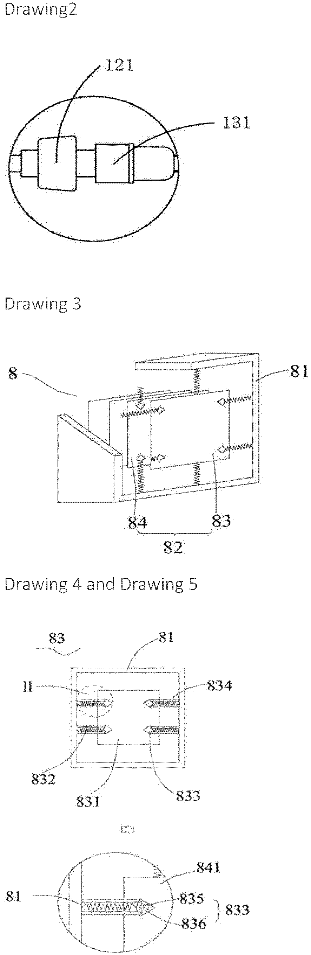

As shown in Drawing 2, compared with the foregoing embodiment, the main line (1) in this embodiment includes the first infusion line (12) and the second infusion line (13); one end of the first infusion line (12) is connected to an infusion needle (11). The other end of the first infusion line (12) is provided with the first tube connector (121); one end of the second infusion line (13) is provided with the second tube connector (131), and the other end of the second infusion line (13) is connected to the first branch line (3) and the second branch line (2); as the first line connector (121) and the second line connector (131) are detachably connected together, the first infusion line (12) and the second infusion line (13) are connected.

In this embodiment, after the separation of the first infusion line (12) and the second infusion line (13), the first infusion line (12) with the infusion needle (11) can be used as a blood taking set in cooperation with the blood collection tube.

### Embodiment 3

As shown in Drawing 1, compared with the foregoing embodiment, the second branch line (2) in this embodiment is also provided with a flow regulator (31), a drip chamber (32) and a filter (33); in the direction from one end of the second branch line (2) connecting the main branch line (2) to the other end of the second branch line (2), the clamp (6), the flow regulator (31), the drip chamber (32) and the filter (33) of the second branch line (2) are installed in sequence.

In this embodiment, the filter (33) is provided with a filter screen, and is connected to the drip chamber (32) through a drip tube, thereby promoting the liquid or blood filtered by the filter screen to enter the drip chamber (32); the flow regulator (31) can change the flow of liquid; the drip chamber (32) can isolate the air in the liquid, thereby promoting the reduction of the harm to the human body caused by the liquid passing through the second branch line.

### Embodiment 4

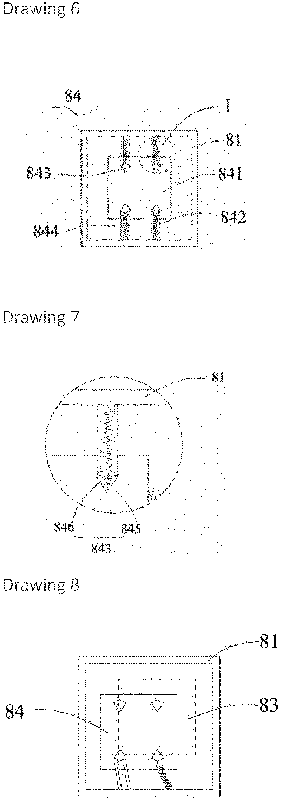

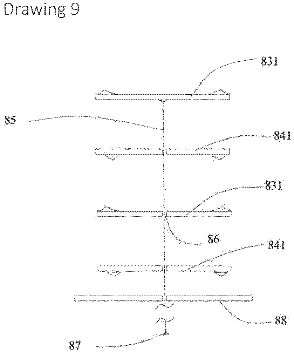

As shown in Drawings 3-9, compared with the foregoing embodiment, in this one, the energy converter (8) consists of an outer frame (81) and a piezoelectric device (82) that converts mechanical energy into electrical energy; the piezoelectric device (82) includes the positive charge induction collector (83) that senses and collects a positive charge and negative charge induction collector (84) that senses and collects a negative charge; the positive charge induction collector (83) and the negative charge induction collector (84) are relatively installed in the outer frame (81), and the positive charge induction collector (83) and the negative charge induction collector (84) is electrically connected to the energy storage device (19). Among which the positive charge induction collector (83) consists of a positive plate (831), a positive pulling structure (832), a positive rectifier anchoring structure (833), and a positive piezoelectric structure (834); the negative charge induction collector (84) consists of a negative plate (841), a negative pulling structure (842), a negative rectifying anchoring structure (843), and the negative piezoelectric structure (844); install the positive rectifier anchoring structure (833) on the positive plate (831); connect one end of the positive pulling structure (832) to the positive rectifier anchoring structure (833), and connect the other end of the positive pulling structure (832) to the outer frame (81); connect one end of the positive piezoelectric structure (834) to the positive rectifier anchoring structure (833), and connect the other end of the positive piezoelectric structure (834) to the outer frame (81); install the negative rectifying anchoring structure (843) on the negative plate (841); one end of the pulling structure (842) is connected to the negative rectifying anchor point structure (842), and the other end of the negative pulling structure (842) is connected to the outer frame (81); one end of the negative piezoelectric structure (844) is connected to the negative rectifying anchor point structure (842), and the other end of the negative piezoelectric structure (844) is connected to the outer frame (81). In this embodiment, the positive plate (831) and the negative plate (841) are made of metal materials. The positive pulling structure (832) and the negative pulling structure (842) are elastic conductor structures, which have strong elasticity and high electrical conductivity, such as metal springs.

When liquid enters the drip chamber, the drip chamber will vibrate. Due to the above-mentioned vibration, the positive plate (831) and the negative plate (841) will move vigorously under the respective pulling of positive pulling structure (832) and negative pulling structure (842), thus driving the positive piezoelectric structure (834) and the negative piezoelectric structure (844) to shake or even twist, which leads them to induce charges. Then to perform charge rectification through the positive rectifier anchoring structure (833) and the negative rectifier anchoring structure (843), so that the positive plate (831) only receives the positive charges induced by the positive piezoelectric structure (834), and the negative plate (841) only receives the negative charges induced by the negative piezoelectric structure (844). With the accumulation of positive charges on the positive plate (831) and the accumulation of negative charges on the negative plate (841), the piezoelectric device (82) converts more and more electric energy. Meanwhile, the air between the positive plate (831) and the negative plate (841), as the dielectric material between the two plates, forms the capacitance structure of positive plate (831) - air dielectric layer - negative plate (841), which realizes the storage function of piezoelectric electric energy. In addition, the electric energy stored in the positive plate (831) and the negative plate (841) is transferred to an external energy storage device.

Moreover, in this embodiment, the positive rectifier anchoring structure (833) consists of a forward diode (835) and a positive anchoring structure (836); the negative rectifying anchoring structure (843) consists of a backward diode (845) and a negative anchoring structure (846); the positive anchoring structure (836) is installed on the positive plate (831); the forward diode (835) is installed on the positive anchoring structure (836); one end of the positive piezoelectric structure (834) is connected to the forward diode (835), the other end of the positive piezoelectric structure (834) is connected to the outer frame (81); one end of the positive pulling structure (832) is connected to the positive anchoring structure (836), the other end of the positive pulling structure (832) is connected to the outer frame (81); the negative anchoring structure (846) is installed on the negative plate (841); the backward diode (845) is installed on the negative anchoring structure (846); one end of the negative piezoelectric structure (844) is connected to the backward diode (845), the other end of the negative piezoelectric structure (844) is connected to the outer frame (81); one end of the negative pulling structure (842) is connected to the negative anchoring structure (846), and the other end of the negative pulling structure (842) is connected to the outer frame (81). In this embodiment, the diode is reversely connected to the negative plate (841) by using the unidirectional conductivity of the diode, which means the negative plate (841) is the cathode of the diode. Forward connect the diode to the positive plate (831), which means the positive plate (831) is the anode of the diode.

In addition, in this embodiment, the positive charge induction collector (83) can be one or more, the same as negative charge induction collector (84). When there are multiple devices, the positive charge induction collector (84) and the negative charge induction collector (84) are installed at intervals. In this embodiment, the installation of multiple positive charge induction collector (83) and negative charge induction collector (84) can further improve the electric energy conversion efficiency of the energy converter (8).

### Embodiment 5

As shown in Drawings 3-9, compared with the foregoing embodiment, in this embodiment, the positive rectifier anchoring structure (833) is installed on both sides of the positive plate (831), and the negative rectifying anchoring structure (843) is installed on both ends of the negative plate (841).

Specifically, the positive rectifier anchoring structure (833) and the negative rectifying anchoring structure (843) are respectively installed on both sides of the positive plate (831) and the two ends of the negative plate (841) in a position of 90°, so that a relative displacement will be generated when the positive plate (831) and the negative plate (841) is respectively pulled by the positive pulling structure (832) and the negative pulling structure (842), which reduces the overlapping area of the positive plate (831) and the negative plate (841), that is, the capacitance area of the positive plate (831) - air dielectric layer - negative plate (841) capacitor structure is reduced. Meanwhile, due to the characteristics of the attraction of positive and negative electric charges, it is concentrated in the area where the positive plate (831) and the negative plate (841) overlap. Then, if the total charge is constant, when increase the output voltage, the energy conversion efficiency of the energy converter (8) will be improved.

### Embodiment 6

As shown in Drawings 3-9, compared with the foregoing embodiment, the energy converter (8) in this embodiment further includes a charge balance structure, which consists of a pulling rope (85), a discharge ring (87), an outer plate (88). Connect the traction rope (85) to the first electrode plate in the outer frame (81), and install the outer plate (88) between the last electrode plate and the discharge ring (87); the remaining electrode plates except the first electrode plate and the outer plate shall be provided with through holes (86), and the traction rope (85) is connected to the discharge ring (87) through the through hole (86). In this embodiment, if there are multiple positive charge induction collectors (83) and multiple negative charge induction collectors (84) in the outer frame, which means there are multiple positive plates (831) and multiple negative plates (841). Positive plates (831) and negative plates (841) are installed at intervals. Taking the positive plate (831) as the first plate as an embodiment, connect one end of the traction rope (85) to the positive plate (831), and connect the other end to the discharge ring (87) by passing through the negative plate (841), the positive plate (831), the negative plate (841)..., and the outer plate (88) in turn.

In this embodiment, install a traction rope (85) on the first electrode plate, and connect it to the discharge ring (87) outside the outer plate (88) by penetrating the other electrode plates. When the positive and negative charges on the positive plate (831) and the negative plate (841) are out of balance, pull the discharge ring (87) to make contact between the positive plate (831) and the negative plate (841) to generate electrical neutralization. The electric charge can be released to the outside by contacting the outer frame (81), so as to reset the charge on the positive plate (831) and the negative plate (841), improving the stability of the electric energy conversion of the energy converter (8).

Although the invention has been shown and described with respect to a preferred embodiment, it is obvious that equivalent alterations and modifications will occur to others skilled in the art upon the reading and understanding of this specification. The present invention includes all such equivalent alterations and modifications, and is limited only by the scope of the claims.

## Claims

1. A blood transfusion set is **characterized by** comprising a main line (1), which is connected with the first branch line (3) and the second branch line (2); the first branch line (3) is connected with the third branch line (4) and the fourth branch line (5); the free end of the main line (1) is provided with an infusion needle (11); the free ends of the second branch line (2), the third branch line (4), and the fourth branch line (5) are all provided with spikes (7); the first branch line (3), the second branch line (2), the third branch line (4) and the fourth branch line (5) are all provided with clamps (6).

2. According to claim 1, the blood transfusion set is **characterized in that** the main line (1) comprises the first infusion line (12) and the second infusion line (13); one end of the first infusion line (12) is connected with an infusion needle (11), and the other end of the first infusion line (12) is provided with the first line connector (121); one end of the second infusion line (13) is provided with the second line connector (131), and the other end of the second infusion line (13) is connected with the first branch line (3) and the second branch line (2).

3. According to claim 2, the blood transfusion set is **characterized in that** the first line connector (121) and the second line connector (131) are detachably connected together; the first infusion line (12) is connected with the second infusion line (13).

4. According to claim 1, the blood transfusion set is **characterized in that** the main line (1), the first branch line (3), the second branch line (2), the third branch line (4) and the fourth branch line (5) are made of PVC material with the same diameter.

5. According to claim 4, the blood transfusion set is **characterized in that** the second branch line (2) is provided with a flow regulator (31), a drip chamber (32) and a filter (33); the clamp (6), flow regulator (31), drip chamber (32) and filter (33) in the second branch line (2) are set in sequence from the end of the second branch line (2) connected with the main line (1) to the other end of the second branch line (2).

6. According to claim 5, the blood transfusion set is **characterized in that** the filter (33) is provided with a filter screen; the filter (33) is connected to the drip chamber (32) through a drip tube.

7. According to claim 1, the blood transfusion set is **characterized in that** the main line (1), the first branch line (3) and the second branch line (2) are connected together through a T valve.

8. According to claim 1, the blood transfusion set is **characterized in that** the first branch line (3), the third branch line (4) and the fourth branch line (5) are connected together through a T valve.

9. According to claim 5 or 6, the blood transfusion set is **characterized in that** the blood transfusion set comprises an illuminated sensor which is detachably provided at the drip chamber (32).

10. According to claim 9, the blood transfusion set is **characterized in that** the illuminated sensor comprises an LED lamp and an energy converter (8); the energy converter (8) is electrically connected to the LED lamp.
